(19) Europäisches Patentamt
European Patent Office
Office européen des brevets

(11) EP 1 140 064 B1

# (12) EUROPEAN PATENT SPECIFICATION

(45) Date of publication and mention of the grant of the patent:
**15.02.2006 Bulletin 2006/07**

(21) Application number: **00910645.1**

(22) Date of filing: **11.01.2000**

(51) Int Cl.:
***A61K 31/337*** *(2006.01)* ***A61K 31/335*** *(2006.01)*
***A61P 35/00*** *(2006.01)*

(86) International application number:
**PCT/EP2000/000943**

(87) International publication number:
**WO 2000/041482 (20.07.2000 Gazette 2000/29)**

## (54) ORAL ADMINISTRATION OF TAXOID DERIVATIVES

ORALE VERABREICHUNG VON TAXOID DERIVATEN

NOUVELLE UTILISATION DE DERIVES DE TAXOIDES

(84) Designated Contracting States:
**AT BE CH CY DE DK ES FI FR GB GR IE IT LI LU MC NL PT SE**

(30) Priority: **13.01.1999 EP 99400072**

(43) Date of publication of application:
**10.10.2001 Bulletin 2001/41**

(73) Proprietor: **Aventis Pharma S.A.**
**92160 Antony (FR)**

(72) Inventor: **BISSERY, Marie-Christine**
**F-94400 Vitry-sur-Seine (FR)**

(74) Representative: **Le Pennec, Magali**
**Aventis Pharma S.A.**
**Direction des Brevets,**
**Tri LEO/144**
**20, avenue Raymond Aron**
**92165 Antony Cedex (FR)**

(56) References cited:
**EP-A- 0 639 577**
**WO-A-00/09120**
**WO-A-96/30355**
**WO-A-97/32869**

- **DICTIONARYBARN: A MEDICAL DICTIONARY**
- **'Cancer Information' UPMC CANCER CENTERS**
- **TRACY O'CONNOR ET AL: 'Development and Cancer' DYNAMIC DEVELOPMENT**
- **ETIENNE J.: 'Biochimie génétique. Biologie moléculaire', 1995, MASSON**
- **DECLARATION OF H. BOUCHARD, NOVEMBER 2001**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 140 064 B1

## Description

The present invention relates to a new use of 4α-acetoxy-2α-benzoyloxy-5β,20-epoxy-1β-hydroxy-7β,10β-dimethoxy-9-oxo-11-taxen-13α-yl (2R,3S)-3-tert-butoxycarbonylamino-2-hydroxy-3-phenyl-propionate for the manufacture of a medicament for use in the treatment by oral administration of tumors.

4α-acetoxy-2α-benzoyloxy-5β,20-epoxy-1β-hydroxy-7β,10β-dimethoxy-9-oxo-11-taxen-13α-yl (2R,3S)-3 tert-butoxycarbonylamino-2-hydroxy-3-phenylpropionate is represented by formula (Ia)

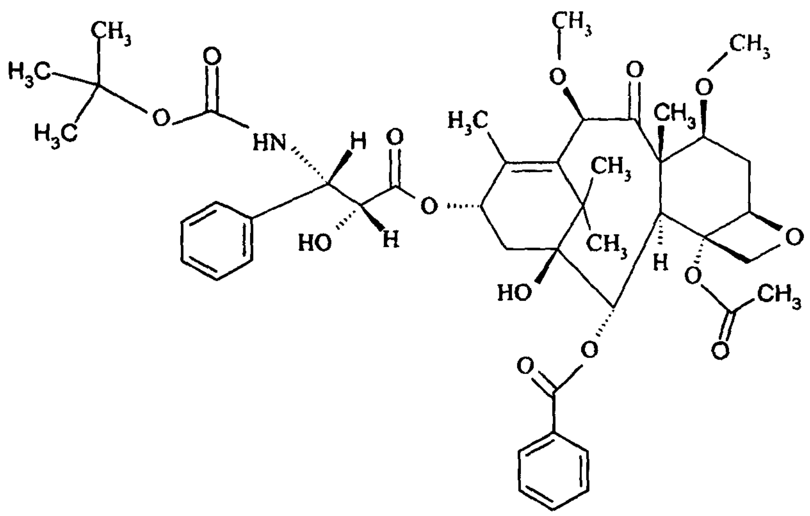

It is known, from patent WO 96/30355, to prepare a derivative according to the present invention by two processes. According to a first, multi-step process, 10-deacetylbaccatin III of formula :

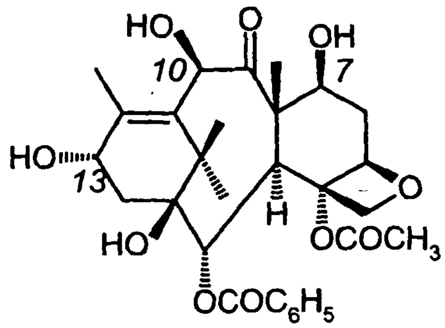

(III)

is selectively protected in positions 7 and 13, for example in the form of a silyl diether, followed by the action of a product of general formula:

R-X (IV)

in which R represents a radical as defined above and X represents a reactive ester residue such as a sulphuric or sulphonic ester residue or a halogen atom, to give a product bearing the unit -OR in position 10 and silyl groups in positions 7 and 13. Next, the silyl protecting groups are replaced with hydrogen atoms to give a compound still bearing the group -OR in position 10 and OH groups in positions 7 and 13. The latter derivative is etherified selectively in position 7 by reaction with the derivative of formula IV to give the derivative of formula (I) in which Z is equal to hydrogen.

The final step consists in esterifying in position 13, according to a process which is known per se, the derivatives of formula (Ia), in which Z represents hydrogen, in the presence of a β-lactam according, for example, to the process described in patent EP 617,018, or in the presence of an oxazolidine as described, for example, in patent WO 96/30355 mentioned above. After deprotection of the protecting groups in positions 7 and 10, an ester of formula (Ia) is thus

obtained in which Z is other than hydrogen and R represents hydrogen. The next step consists in reacting the positions 7 and 10 simultaneously by the action of a reagent formed in situ from a sulphoxide of formula (V) and acetic anhydride (Pummerer-type reaction),

R-SO-R (V)

in which R has the same meaning as above, to form an alkylthioalkyloxy-type intermediate on positions 7 and 10.

The final step, which allows the desired compound of formula (Ia) to be obtained, is carried out on the intermediate compound obtained above, by the action of activated Raney nickel.

Generally, the action of the reagent formed in situ from sulphoxide of general formula (V), preferably dimethyl sulphoxide and acetic anhydride, is carried out in the presence of acetic acid or an acetic acid derivative such as a haloacetic acid, at a temperature of between 0 and 50°C.

Generally, the action of the activated Raney nickel in the presence of an aliphatic alcohol or an ether is carried out at a temperature of between -10 and 60°C.

In the FR 97-14442 application a further process has been described. This invention allows, in a single step, the direct, selective and simultaneous alkylation of the two hydroxyl functions in positions 7 and 10 of 10-deacetylbaccatin or of derivatives thereof esterified in position 13, of formula (VI)

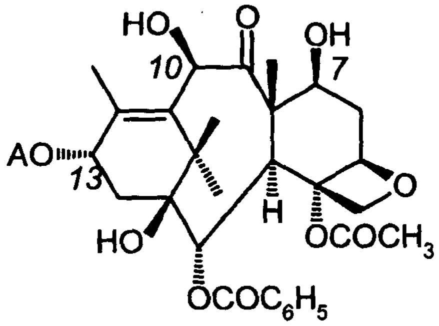

(VI)

in which A represents hydrogen or a side chain of formula (IIa) below :

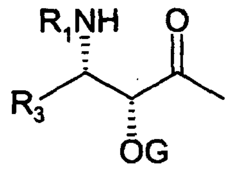

(IIa)

in which G represents a protecting group for the hydroxyl function, $R_1$ and $R_3$ have the same meaning as in formula (II) or an oxazolidine unit of formula (Id) :

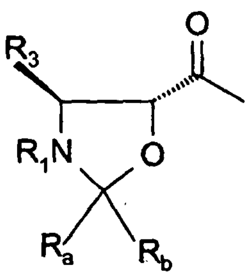

(IIb)

in which $R_1$ and $R_3$ have the same meaning as in formula (II), $R_a$ and $R_b$, which may be identical or different, represent hydrogen or alkyl, aryl, halo, alkoxy, arylalkyl, alkoxyaryl, haloalkyl, haloaryl, it being possible for the substituents optionally to form a 4- to 7-membered ring.

It is preferred to use 10-deacetylbaccatin as starting material, i.e. the product of formula (III), which allows appreciable economy as regards the process and moreover avoids the intermediate protection and deprotection steps necessary in the old processes.

Among the groups G for protecting the hydroxyl function of formula (IIa), it is generally preferred to choose all of the protecting groups described in books such as Greene and Wuts, Protective Groups in Organic Synthesis, 1991, John Wiley & Sons, and MacOmie, Protective Groups in Organic Chemistry, 1975, Plenum Press, and which are deprotected under conditions which degrade the rest of the molecule little or not at all, such as, for example:

- ethers, and preferably ethers such as methoxymethyl ether, 1-ethoxyethyl ether, benzyloxymethyl ether, p-methoxybenzyloxymethyl ether, benzyl ethers optionally substituted with one or more groups such as methoxy, chloro, nitro, 1-methyl-1-methoxyethyl ether, 2-(trimethylsilyl)ethoxymethyl ether, tetrahydropyranyl ether and silyl ethers such as trialkylsilyl ethers,
- carbonates such as trichloroethyl carbonates.

More particularly, the radicals $R_a$ and $R_b$ of general formula (IIb) are chosen from those described in patent WO 94/07878 and the derivatives more particularly preferred are those in which $R_a$ is hydrogen and $R_b$ is a p-methoxyphenyl radical.

The alkylating agent is chosen from :

- alkyl halides, and preferably from alkyl iodides (RI)
- alkyl sulphates such as methyl sulphate
- oxoniums such as trialkyloxonium boric salts, in particular trimethyloxonium tetrafluoroborate ($Me_3OBF_4$).

Methyl iodide is preferably used.

The alkylating agent is used in the presence of an anionization agent such as one or more strong bases, in anhydrous medium.

Among the bases which can be used in anhydrous medium, mention may be made of :

- alkali metal hydrides such as sodium or potassium hydride
- alkali metal alkoxides such as potassium tert-butoxide
- silver oxide $Ag_2O$
- 1,8-bis(dimethylamino)naphthalene
- mono- or dimetallic base mixtures such as those described, for example, in publications such as P. Caubère Chem. Rev. 1993, 93, 2317-2334 or M. Schlosser Mod. Synth. Methods (1992), 6, 227-271; in particular the alkyllithium/alkali metal t-butoxide or alkali metal amide/alkali metal t-butoxide combinations are preferred. One of the two bases can be generated "in situ".

Among all of the possible combinations of alkylating agent and anionization agent, it is preferred to use methyl iodide in the presence of potassium hydride.

The reaction is preferably carried out in an organic medium which is inert under the reaction conditions. Among the solvents, it is preferred to use :

- ethers such as tetrahydrofuran or dimethoxyethane
- when silver oxide is used, it is preferred to use polar aprotic solvents such as dimethylformamide, or aromatic solvents such as toluene
- when 1,8-bis(dimethylamino)naphthalene is used, it is preferred to use alkylesters such as ethylacetate.

For better implementation of the invention, it is preferred to use a molar ratio between the anionization agent and the substrate of greater than 2 and preferably between 2 and 20.

It is also preferred to use a molar ratio between the alkylating agent and the substrate of greater than 2 and preferably between 2 and 40.

It is preferred to use a reaction temperature of between -30°C and 80°C.

The reaction time advantageously ranges between a few hours and 48 hours depending on the reagents chosen.

After the alkylating step, when the latter is carried out on 10-deacetylbaccatin, the process then proceeds, in a known manner, to the esterification step according, for example, to the processes described in patents EP 617,018 or WO 96/30355 mentioned above.

Thus, according to a first, 3-step process, the procedure first begins with the dialkylation of 10-deacetylbaccatin, using an alkylating agent in the presence of a strong base, in a second step, the 10-deacetylbaccatin dietherified in

positions 7 and 10 is coupled, in position 13, with a suitably protected β-lactam in the presence of an activating agent chosen from tertiary amines and metal bases which ensure the formation of an alkoxide in position 13. Deprotection of the side chain is then achieved by the action of an inorganic or organic acid.

Thus, according to a second, 3-step process, the procedure first begins with the dialkylation of 10-deacetylbaccatin, using an alkylating agent in the presence of a strong base, in a second step, the 10-deacetylbaccatin dietherified in positions 7 and 10 is coupled, in position 13, with an oxazolidine in the presence of a coupling agent such as diimides in the presence of an activating agent such as dialkylaminopyridines. Opening of the oxazolidine is achieved by the action of an inorganic or organic acid.

According to a third process, the procedure begins with the esterification in position 13 ofbaccatin suitably protected in positions 7 and 10, with a β-lactam or an oxazolidine in the presence of a coupling agent and/or an activating agent as described in the above two processes. After deprotection in positions 7 and 10, the dietherification in positions 7 and 10 is carried out by an alkylating agent in the presence of a strong base. Deprotection of the side chain is then achieved by the action of an inorganic or organic acid.

Formulations suitable for oral administration means formulations which are in a form suitable to be administered orally to a patient. The formulations may be presented as any suitable oral dosage form such as for example capsules, cachets or tablets each containing a predetermined amount of the active ingredient; as a powder or granules; as solution or a suspension in an aqueous liquid or a non-aqueous liquid; or as an oil-in-water liquid emulsion or a water-in-oil liquid emulsion. The active ingredient may also be presented as a bolus, electuary or paste.

Pharmaceutical composition means a composition comprising the compound of formula (Ia) and at least one component selected from the group comprising pharmaceutically acceptable carriers, diluents, adjuvants, excipients, or vehicles, such as preserving agents, fillers, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms. Examples of suspending agents include ethoxylated isostearyl alcohols, polyoxyethylene sorbitol and sorbitan esters, microcrystalline cellulose, aluminum metahydroxide, bentonite, agar-agar and tragacanth, or mixtures of these substances. Prevention of the action of microorganisms can be ensured by various antibacterial and antifungal agents, for example, parabens, chlorobutanol, phenol, sorbic acid, and the like. Examples of suitable carriers, diluents, solvents or vehicles include water, ethanol, polyols, suitable mixtures thereof, vegetable oils (such as olive oil) and organic esters such as ethyl oleate. Examples of excipients include lactose, milk sugar, sodium citrate, calcium carbonate, dicalcium phosphate. Examples of disintegrating agents include starch, alginic acids and certain complex silicates. Examples of lubricants include magnesium stearate, sodium lauryl sulphate, talc, as well as high molecular weight polyethylene glycols.

Liquid dosage form means the dose of the active compound to be administered to the patient is in liquid form, for example, pharmaceutically acceptable emulsions, solutions, suspensions, syrups and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, castor oil and sesame oil, glycerol, tetrahydrofurfuryl alcohol, polyethylene glycols, fatty acid esters of sorbitan and phospholipids or mixtures of these substances, and the like.

Solid dosage form means the dosage form of the compound useful according to the invention is solid form, for example capsules, tablets, pills, powders, dragees or granules. In such solid dosage forms, the compound useful according to the invention is admixed with at least one inert customary excipient (or carrier) such as sodium citrate or dicalcium phosphate or (a) fillers or extenders, as for example, starches, lactose, sucrose, glucose, mannitol and silicic acid, (b) binders, as for example, carboxymethylcellulose, alignates, gelatin, polyvinylpyrrolidone, sucrose and acacia, (c) humectants, as for example, glycerol, (d) disintegrating agents, as for example, agar-agar, calcium carbonate, potato or tapioca starch, alginic acid, certain complex silicates and sodium carbonate, (e) solution retarders, as for example paraffin, (f) absorption accelerators, as for example, quaternary ammonium compounds, (g) wetting agents, as for example, cetyl alcohol and glycerol monostearate, (h) adsorbents, as for example, kaolin and bentonite, (i) lubricants, as for example, talc, calcium stearate, magnesium stearate, solid polyethylene glycols, sodium lauryl sulfate, (j) opacifying agents, (k) buffering agents , and agents which release the compound(s) useful according to the invention in a certain part of the intestinal tract in a delayed manner.

The invention will be more accurately defined by the following examples.

Product of formula (Ia) is formulated as following :

Formulation II : 0,2 mg/ml of product of formula Ia in a solution containing 5 % ethanol, 5 % Polysorbate 80 and 90 % of an aqueous 5 % glucose solution. 15 female C3H/HeN mice each weighing ca 20 g were each administered orally with formulated product II to give a dose of 20.6 $mg.kg^{-1}$ per administration per mouse, 12,8 ; 8,4 and 5,5 each day for 5 days.

Animals : The animals subjected to the experiment, generally mice, are subcutaneously grafted bilaterally with 30 to 60 mg of a mammary tumor fragment (adenocarcinoma MA17/A) on day 0. The animals bearing tumors are mixed

before being assigned to the various treatments and controls groups. Chemotherapy generally begins from 3 to 7 days after grafting, depending on the type of tumour, and the animals are observed every day. The different animal groups are weighed 3 or 4 times a week until the maximum weight loss is attained, and the groups are then weighed at least once a week until the end of the trial.

Efficacy : The efficacy of a new pharmaceutical form may be evaluated by:
- the tumor growth inhibitor (T/C)

The T/C value in percent is an indication of antitumor effectiveness :

$$T/C\ (\%) = \frac{\text{Median tumor weight of the Treated}}{\text{Median tumor weight of the Control}} \times 100$$

According to NCI standards, a T/C < 42 % is the minimal level to declare activity. A T/C < 10 % is considered to indicate high antitumor activity and is the level used by NCI to justify further development (Decision Network-2 level, DN-2). The T/C value can be converted to an arbitrary activity rating according to the NCI criteria:

| NCI activity | % T/C |
|---|---|
| Highly active ++ | ≤ 10 |
| Active + | 11-42 |
| Inactive - | > 42 |

- and quantified by the log cell kill which is determined

$$\log_{10} \text{ cells killed} = \text{T-C (days)} / 3.32 \times T_d$$

in which T-C represents the time in days for the tumours of the treated group (T) and the tumors of the non-treated group (C) to reach a predetermined value (750 mg for example) [T.H. CORBETT et al., Cancer, 40, 2660-2680 (1977) ; F.M. SCHABEL et al., Cancer Drug Development, Part B. Methods in Cancer Research, 17, 3-51, New York, Academic Press Inc. (1979)]. A product is considered to be active if $\log_{10}$ cells killed is greater than or equal to 0.7. A product is considered to be very active if $\log_{10}$ cells killed is greater than 2.8.

Results :

| Agent | Route volume | Dosage in mg/kg/dose | Schedule in days | Total dose in mg/kg | Drug death (days of death) | Average body weight change in % per mouse post last treatment (day) | Median tumor weight in mg on day 12 (range) | T/C in % day 12 | Time for median tumor to reach 750 mg in days | T-C in days | log cell kill | Comments |
|---|---|---|---|---|---|---|---|---|---|---|---|---|
| Product of formula Ia | p.o. | 20.6 | 3 to 7 | 103.0 | 5/5 (7,2d8,10,11) | - | - | - | - | - | - | Toxic |
| | 0.2 ml | 12.8 | | 64.0 | 0/5 | -17.9 (10) | 0 (0-0) | 0 | 25.9 | 13.8 | 3.2 | HNTD higly active |
| | | 8.4 | | 42.0 | 0/5 | -11.0 (9) | 0 (0-268) | 0 | 18.2 | 6.1 | 1.4 | Active |
| | | 5.5 | | 27.5 | 0/5 | +0.2(8 | 271 (107-432) | 38 | 14.4 | 2.3 | 0.5 | Inactive |
| Control | | | | | | | 704 (312-864) | | 12.1 | | | |

Tumor doubling time = 1.3 days 10 mice in the control group. Mice average weight (product of formula Ia) = 20.39 g

Abbreviation used : HNTD = highest nontoxic dose

Comments : Product of formula Ia was evaluated against early stage mammary adenocarcinoma MA17/A using a daily schedule for 5 days. At the highest nontoxic dose 12.8 mg/kg/administration (total dose 64 mg/kg), product Ia was found highly active in this tumor model with a 0 % T/C and a 3.2 log cell kill total.

## Claims

1. Use of 4α-acetoxy-2α-benzoyloxy-5β,20-epoxy-1β-hydroxy-7β,10β-dimethoxy-9-oxo-11-taxen-13α-yl (2R,3S)-3-tert-butoxycarbonylamino-2-hydroxy-3-phenyl-propionate for the manufacture of a medicament for use in the treatment by oral administration of tumors.

## Patentansprüche

1. Verwendung von 4α-acetoxy-2α-benzoyloxy-5β,20-epoxy-1β-hydroxy-7β,10β-dimethoxy-9-oxo-11-taxen-13α-yl (2R,3S)-3-tert-butoxycarbonylamino-2-hydroxy-3-phenyl-proprionat zur Herstellung eines Medikaments zur Verwendung in der Behandlung von Tumoren durch orale Verabreichung.

## Revendications

1. Utilisation du 4α-acétoxy-2α-benzoyloxy-5β,20-époxy-1β-hydroxy-7β,10β-diméthoxy-9-oxo-11-taxén-13α-yl-(2R,3S)-3-tert-butoxycarbonylamino-2-hydroxy-3-phénylpropionate pour la fabrication d'un médicament destiné à être utilisé pour le traitement de tumeurs en administration par voie orale.